# EUROPÄISCHE PATENTANMELDUNG

(11) **EP 2 520 221 A1**
(43) Veröffentlichungstag der Anmeldung: **07.11.2012**
(21) Anmeldenummer: 11164622.0
(22) Anmeldetag: 03.05.2011
(51) Int. Cl.: A61B 5/00, A61B 1/24, A61B 18/20, A61B 18/22, A61C 19/04, A61C 1/08

(54) **Handstück für ein medizinisches Diagnosegerät und medizinisches Diagnosegerät**

(71) Anmelder: W & H Dentalwerk Bürmoos GmbH, 5111 Bürmoos (AT)
(72) Erfinder: Kasenbacher, Anton, 83278, Traunstein (DE); Heinrich, Christoph, 5110, Oberndorf (AT); Irran, Thomas, 5020, Salzburg (AT)
(74) Vertreter: Patentanwälte Lambsdorff & Lange

(57) **Zusammenfassung**

Das Handstück (40) weist einen Handstück-Innenraum (40.2) auf, in welchem eine Strahlung (45) in Richtung auf ein Handstückende führbar ist, wobei das Handstück (40) eine oder mehrere optische Erfassungsvorrichtungen (44) aufweist, welche jeweils mindestens teilweise im Handstück-Innenraum (40.2) angeordnet sind.

## Beschreibung

Die vorliegende Erfindung betrifft ein Handstück für ein medizinisches Diagnosegerät und ein medizinisches Diagnosegerät gemäß den unabhängigen Patentansprüchen.

Handstücke für medizinische Diagnosegeräte werden in unterschiedlichen Bereichen und zu unterschiedlichen Zwecken in mannigfaltigen Formen eingesetzt. Ein Diagnosegerät kann dabei beispielsweise in Form eines optischen Diagnosegeräts, insbesondere als Laser-Diagnosegerät, ausgeführt werden. Ein Diagnosegerät kann andererseits auch zusätzlich als ein Bearbeitungsgerät, etwa in Form eines Ablationsgerätes, ausgeführt werden, wobei hier insbesondere im Dentalbereich die Kariesdiagnose und anschließende Ablation von Bedeutung ist. Ein Handstück für ein derartiges medizinisches Diagnosegerät ist so auszugestalten, dass die Diagnose unter optimalen Bedingungen durchgeführt werden kann und gegebenenfalls zusätzlich auch eine anschließende Bearbeitung, wie eine Ablation mit einem Laserstrahl gleichermaßen optimal erfolgen kann.

Es ist daher Aufgabe der vorliegenden Erfindung, ein Handstück für ein medizinisches Diagnosegerät sowie ein medizinisches Diagnosegerät bereitzustellen, mit welchen eine medizinische Diagnose, wie etwa eine Erkennung von Karies an Zähnen, unter optimalen Bedingungen erfolgen kann.

Diese Aufgabe wird durch ein Handstück für ein medizinisches Diagnosegerät und ein medizinisches Diagnosegerät gemäß den unabhängigen Patentansprüchen gelöst. Vorteilhafte Weiterbildungen und Ausgestaltungen sind Gegenstand von Unteransprüchen.

Ein erfindungsgemäßes Handstück für ein medizinisches Diagnosegerät weist einen Handstück-Innenraum auf, in welchem eine Strahlung in Richtung auf ein Handstückende führbar ist. Das Handstück weist des Weiteren eine oder mehrere optische Erfassungsvorrichtungen auf, welche jeweils mindestens teilweise im Handstück-Innenraum angeordnet sind.

Die Erfindung geht davon aus, dass mittels einer Strahlungsquelle eine Strahlung einer vorgegebenen Wellenlänge ausgesendet und mittels eines dafür geeigneten Handstücks auf einen zu diagnostizierenden Bereich, wie etwa einen Zahn, appliziert werden soll. Eine wesentliche Erkenntnis der Erfindung besteht darin, dass der optische Aufbau bisher verwendeter oder vorgeschlagener Handstücke ungünstig ist, da die von der Zahn- oder Gewebeoberfläche reflektierte oder zurückgeworfene Strahlung auf demselben Lichtpfad, auf dem eine Diagnosestrahlung zu dem Zahn geführt wurde, aus dem Handstück geführt und außerhalb des Handstücks detektiert wird. Die Erfindung schafft demgegenüber Abhilfe, indem sie vorsieht, dass eine oder mehrere optische Erfassungsvorrichtungen für die von dem Zahn reflektierte oder zurückgeworfene Strahlung innerhalb des Handstück-Innenraums vorgesehen sind. Damit kann die von dem Zahn reflektierte oder zurückgeworfene Strahlung entweder bereits innerhalb des Handstücks detektiert werden oder zumindest innerhalb des Handstücks durch dafür vorgesehene Lichtwellenleiter erfasst werden.

Gemäß einer Ausführungsform des Handstücks ist in dessen Handstück-Innenraum eine erste Umlenkvorrichtung, welche vorzugsweise durch einen Spiegel gegeben ist, zur Umlenkung der Strahlung in Richtung auf das Handstückende angeordnet. Die erste Umlenkvorrichtung kann beispielsweise innerhalb eines Winkelstücks angeordnet sein, welches mit einem longitudinalen Abschnitt des Handstücks verbunden ist und an dessen einem Ende sich das Handstückende befindet. Die erste Umlenkvorrichtung lenkt dann die Strahlung aus einer Längsachse des longitudinalen Abschnitts in eine dazu querverlaufende Achse in Richtung auf das Handstückende um. Die Strahlung kann eine Diagnosestrahlung oder eine Bearbeitungsstrahlung sein. Die erste Umlenkvorrichtung kann somit gleichermaßen für die Umlenkung einer Diagnosestrahlung wie auch einer Bearbeitungsstrahlung auf einen Gewebeabschnitt verwendet werden. Die Strahlung selbst kann eine monochromatische, kohärente Strahlung, also beispielsweise eine Laserstrahlung, oder eine spektral breitbandige, inkohärente Strahlung sein. Die Strahlungsquellen können innerhalb wie außerhalb des Handstücks angeordnet sein. Insbesondere falls die Strahlungsquellen durch Laserdioden gebildet werden, können diese innerhalb des Handstücks angeordnet sein.

Die optische Erfassungsvorrichtung ist somit vorzugsweise für die Erfassung von am Handstückende in den Handstück-Innenraum eintretenden optischen Signalen ausgelegt. Diese optischen Signale können je nach Diagnoseverfahren beispielsweise eine zweite Harmonische der auf das Gewebe eingestrahlten Laserstrahlung, eine aufgrund der auf das Gewebe eingestrahlten Strahlung erzeugte Fluoreszenzstrahlung oder auch eine von dem Gewebe reflektierte Strahlung sein. Demzufolge kann die optische Erfassungsvorrichtung für die Erfassung und Detektion von optischen Signalen eines ersten vorgegebenen Wellenlängenbereichs ausgelegt sein, bzw. eine von mehreren optischen Erfassungsvorrichtungen können für die Erfassung und Detektion von optischen Signalen eines ersten vorgegebenen Wellenlängenbereichs ausgelegt sein und eine weitere optische Erfassungsvorrichtung kann für die Erfassung und Detektion von optischen Signalen eines zweiten vorgegebenen Wellenlängenbereichs ausgelegt sein.

Gemäß einer Ausführungsform kann die optische Erfassungsvorrichtung in einem Strahlengang der optischen Signale eine zweite Umlenkvorrichtung, eine Linse und einen optischen Sensor aufweisen, wobei die genannten optischen Elemente im Strahlengang in der genannten Reihenfolge angeordnet sein können. Für den Fall des Vorhandenseins weiterer optischer Erfassungsvorrichtungen können diese ebenfalls jeweils eine Umlenkvorrichtung, eine Linse und einen optischen Sensor aufweisen. Insbesondere kann dabei vorgesehen sein, dass eine die Linse und den optischen Sensor aufweisende Einheit in einer Strahlrichtung der optischen Signale linear verschiebbar ausgelegt ist. Dies ermöglicht es, die optische Erfassungsvorrichtung als autofokusgesteuerte optische Erfassungsvorrichtung auszulegen, bei der durch die lineare Verschiebung sichergestellt ist, dass stets die von einer Oberfläche des zu diagnostizierenden Gewebes reflektierte oder zurückgeworfene Strahlung detektiert wird.

Gemäß einer Ausführungsform kann die zweite Umlenkvorrichtung, welche vorzugsweise durch einen Spiegel gegeben ist, in sich verschwenkt oder verkippt werden, insbesondere also um eine durch sie selbst hindurchtretende Achse schwenkbar gehaltert sein, insbesondere um zwei aufeinander senkrechte, durch sie selbst hindurchtretende Achsen schwenkbar gehaltert sein. Damit ist es möglich, optische Signale von verschiedenen Punkten der zu diagnostizierenden Gewebeoberfläche zu erfassen und zu detektieren. Dies kann insbesondere dann der Fall sein, wenn der Fokus der auf das Gewebe gerichteten Diagnosestrahlung mittels einer Scan-Einheit über die Gewebeoberfläche gerastert wird, sodass in diesem Fall der optischen Erfassungsvorrichtung ermöglicht wird, in gleicher Weise die Oberfläche des Gewebes synchron mit der Diagnosestrahlung abzuscannen oder abzutasten.

Gemäß einer Ausführungsform ist die optische Erfassungsvorrichtung ferner im Strahlengang der optischen Signale nach der ersten Umlenkvorrichtung angeordnet oder anordenbar. In diesem Fall treten die optischen Signale durch die erste Umlenkvorrichtung hindurch und treten anschließend in die optische Erfassungsvorrichtung ein, sodass vorgesehen ist, dass die erste Umlenkvorrichtung für die Strahlung der optischen Signale durchlässig ist.

Gemäß einer Ausführungsform kann auch vorgesehen sein, dass die erste Umlenkvorrichtung zur Umlenkung des Laserstrahls aus einer longitudinalen Achse oder Längsachse des Handstücks in eine Querachse ausgelegt ist und die optische Erfassungsvorrichtung um die Längsachse schwenkbar ist. Insbesondere kann zu diesem Zweck die optische Erfassungsvorrichtung an einer, innerhalb des Winkelstücks angeordneten revolverartigen Schwenkvorrichtung befestigbar oder befestigt sein. An einer derartigen Schwenkvorrichtung können dann gegebenenfalls auch eine oder mehrere weitere optische Erfassungsvorrichtungen befestigbar oder befestigt sein. Diese können aber auch auf andere Art und Weise innerhalb des Handstück-Innenraums angeordnet sein.

Gemäß einer Ausführungsform kann eine gegebenenfalls vorgesehene weitere optische Erfassungsvorrichtung für die Erfassung und Detektion von optischen Signalen eines vorgegebenen Wellenlängenbereichs ausgelegt sein, welcher von dem ersten vorgegebenen Wellenlängenbereich der ersten optischen Erfassungsvorrichtung verschieden ist. Beispielsweise kann die erste optische Erfassungsvorrichtung auf die Erfassung und Detektion der von einem Gewebe zurückgeworfenen zweiten Harmonischen der auf das Gewebe eingestrahlten Laserstrahlung ausgelegt sein, während eine zweite oder weitere optische Erfassungsvorrichtung auf die Erfassung der von einem Gewebe aufgrund optischer Anregung erzeugte und zurückgeworfene Fluoreszenzstrahlung ausgelegt sein kann, wobei die Fluoreszenzstrahlung in einem bestimmten vorgegebenen Wellenlängenbereich liegt.

Gemäß einer Ausführungsform kann die optische Erfassungsvorrichtung auch für die Erfassung und Detektion von optischen Signalen im UV-Bereich ausgelegt sein. Dies kann insbesondere der Fall sein, wenn mittels eines Bearbeitungs- wie eines Ablations-Laserstrahls ein Mikroplasma auf der Gewebeoberfläche gezündet wird, durch welches UV-Strahlung erzeugt wird, die mittels der optischen Erfassungsvorrichtung erfasst und detektiert werden kann.

Gemäß einer Ausführungsform kann die optische Erfassungsvorrichtung einen optischen Lichtwellenleiter aufweisen, welcher mit seinem lichteintrittsseitigen Ende auf das Handstückende ausgerichtet ist. In diesem Fall beschränkt sich die Funktion der optischen Erfassungsvorrichtung darauf, die von einem Gewebe zurückgeworfene oder reflektierte Strahlung geeignet zu sammeln und mittels des optischen Lichtwellenleiters zu einem Detektor zu führen, der gegebenenfalls auch außerhalb des Handstücks bzw. Handstück-Innenraums angeordnet sein kann. Dies kann beispielsweise der Fall sein, wenn die Diagnose mittels einer optischen Kohärenztomographie-(OCT-)Einrichtung erfolgt, welche größtenteils außerhalb des Handstücks angeordnet ist und bei welcher mittels eines oder mehrerer Lichtwellenleiter Strahlung von und zu dem zu untersuchenden Gewebe geführt wird.

Ein erfindungsgemäßes medizinisches Diagnosegerät weist eine oder mehrere Strahlungsquellen zur Aussendung von Strahlung von jeweils einer vorgegebenen Wellenlänge auf einen zu diagnostizierenden Bereich eines Gewebes auf. Ferner weist das medizinische Diagnosegerät ein wie oben beschriebenes erfindungsgemäßes Handstück auf. Schließlich weist das medizinische Diagnosegerät noch eine Auswerteeinrichtung auf, welche mit der einen oder den mehreren optischen Erfassungsvorrichtungen verbunden ist, welche in dem Handstück bzw. dem Handstück-Innenraum angeordnet sind.

Gemäß einer Ausführungsform weist die Strahlungsquelle oder eine der Strahlungsquellen des Diagnosegeräts eine Laserstrahlquelle auf. Die optische Erfassungsvorrichtung kann dann einen optischen Sensor aufweisen, welcher für die Detektion von der von dem Gewebe zurückgeworfener Strahlung der zweiten Harmonischen der Laserstrahlung ausgelegt ist.

Gemäß einer Ausführungsform kann die Strahlungsquelle oder eine der Strahlungsquellen des Diagnosegeräts eine monochromatische Strahlung emittieren und die optische Erfassungsvorrichtung kann dann einen optischen Sensor aufweisen, welcher für die Detektion von von dem Gewebe zurückgeworfenen Fluoreszenzstrahlung ausgelegt ist.

Gemäß einer Ausführungsform des medizinischen Diagnosegeräts kann die optische Erfassungsvorrichtung einen optischen Sensor aufweisen, welcher für die Detektion von UV-Strahlung ausgelegt ist.

Gemäß einer Ausführungsform kann das Diagnosegerät eine Kohärenztomographie-(OCT-)Einrichtung aufweisen. Insbesondere kann dabei die optische Erfassungsvorrichtung einen optischen Lichtwellenleiter aufweisen, welcher ein Teil der Kohärenztomographie-Einrichtung ist und mit seinem lichteintrittsseitigen Ende auf das Handstückende ausgerichtet ist.

Im Folgenden wird die Erfindung in Form von Ausführungsbeispielen anhand der Zeichnungen näher erläutert. Es zeigen:
- Fig. 1: eine schematische Querschnitts-Darstellung eines medizinischen Diagnosegeräts gemäß einer Ausführungsform;
- Fig. 2: eine schematische Querschnitts-Darstellung eines Handstücks gemäß einer Ausführungsform;
- Fig. 3: eine schematische Querschnitts-Darstellung eines endseitigen Abschnitts eines Handstücks gemäß einer Ausführungsform; und
- Fig. 4: eine schematische Querschnitts-Darstellung eines Handstücks gemäß einer Ausführungsform und ein Blockschaltbild einer daran angeschlossenen Auswerteeinrichtung.

Die Elemente der Zeichnungen sind nicht oder nicht notwendigerweise maßstabsgetreu. Die Merkmale der verschiedenen Ausführungsbeispiele können auch untereinander ausgetauscht werden.

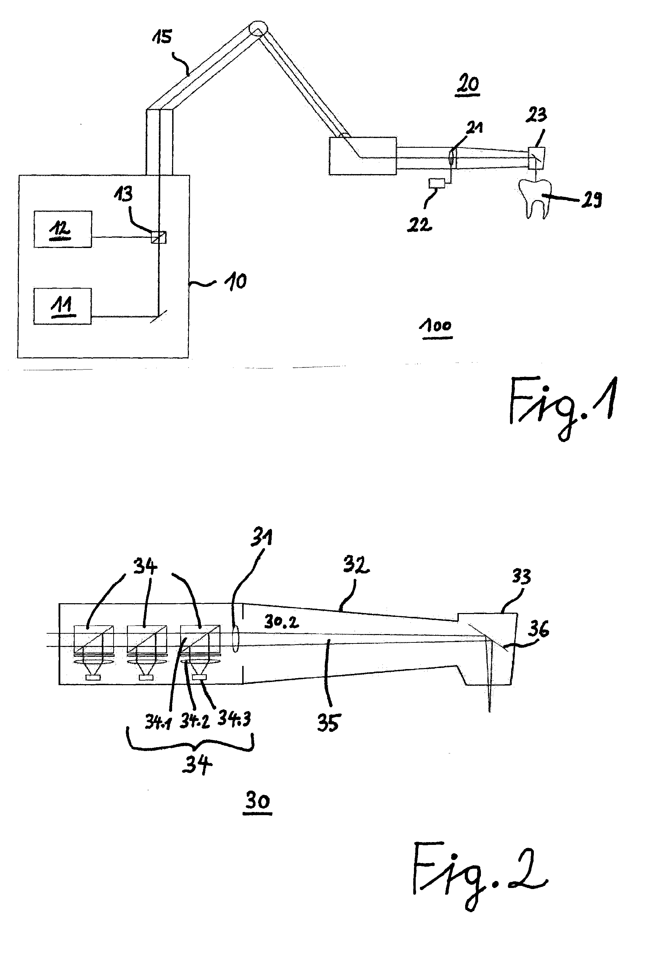

In der Fig. 1 ist ein medizinisches Diagnosegerät schematisch dargestellt. Wie noch erläutert werden wird, kann das Diagnosegerät dabei gleichzeitig ein Behandlungsgerät sein, mit welchem eine zuvor diagnostizierte Zahnkaries mittels eines Laserbearbeitungsstrahls entfernt bzw. ablatiert werden kann. Das Diagnosegerät 100 weist eine Steuer- und Auswerteeinrichtung 10 auf, in welcher eine Laserstrahlquelle 11 und eine Auswerteeinrichtung 12 enthalten sind. Die Laserstrahlquelle 11 emittiert einen Laserstrahl, welcher zwischen einem Bearbeitungsmodus und einem Diagnosemodus hin- und hergeschaltet werden kann, wobei in dem Bearbeitungsmodus die Energie des Laserstrahls so eingestellt werden kann, dass mit dem Laserstrahl eine Ablation erfolgen kann, während in dem Diagnosemodus die Energie des Laserstrahls so eingestellt werden kann, dass sie unterhalb einer Ablationsschwelle verbleibt und mit ihr lediglich eine Diagnosefunktion erfüllt werden kann.

Die Steuer- und Auswerteeinrichtung 10 ist mit einem mehrteiligen Gelenkarm 15 verbunden, welcher mehrere, durch Gelenke miteinander verbundene, lineare Elemente aufweist, in denen der Laserstrahl oder die von einem Zahn 29 zurückgekoppelte Strahlung geführt und an den Gelenken geeignet umgelenkt werden kann. Der Gelenkarm ist an einem Ende mit einem Handstück 20 verbunden, an dessen Ende ein Winkelstück 23 angekoppelt ist, innerhalb dem der Laserstrahl auf einen Zahn 29 umgelenkt werden kann. Innerhalb des Handstücks 20 befindet sich außerdem eine linear in Strahlrichtung verschiebbare Linse 21, mit welcher der Laserstrahl auf den Zahn 29 fokussiert wird. Die Linse 21 wird von einer Autofokus-Steuervorrichtung 22 derart angesteuert, dass sichergestellt ist, dass der Fokus des Laserstrahls stets auf der Oberfläche des Zahns 29 positioniert ist. Die von dem Zahn 29 zurückgeworfene oder reflektierte Strahlung kann an einem in der Steuer- und Auswertevorrichtung 10 enthaltenen Strahlteiler 13 ausgekoppelt und in der Auswerteeinrichtung 12 ausgewertet werden.

Das Handstück 20 ist erfindungsgemäß so ausgebildet, dass eine oder mehrere optische Erfassungsvorrichtungen (nicht dargestellt) mindestens teilweise im Handstück-Innenraum angeordnet sind. In dem Diagnosegerät der Fig. 1 wird zumindest ein Strahlungsanteil oder ein spektraler Anteil der von dem Zahn 29 zurückgeworfenen oder reflektierten Strahlung außerhalb des Handstücks 20, nämlich an dem außerhalb des Handstücks 20 befindlichen Strahlteiler 13 abgelenkt und der Auswerteeinrichtung 12 zugeführt, in der ein Sensor zur Erfassung der Strahlung enthalten sein kann. Im Folgenden wird anhand von Ausführungsbeispielen gezeigt, wie das Erfassen und gegebenenfalls auch Detektieren der von dem Zahn 29 zurückgeworfenen Strahlung innerhalb des Handstücks 20 durchgeführt werden kann.

Die Fig. 2 zeigt eine schematische Querschnitts-Darstellung eines Handstücks gemäß einer Ausführungsform. Das Handstück 30 weist einen longitudinalen Abschnitt 32 auf, an dessen einem Ende ein Winkelstück 33 angefügt ist. In dem Handstück-Innenraum 30.2 wird ein Laserstrahl 35 geführt, welcher durch eine Linse 31 auf eine Gewebeoberfläche wie eine Zahnoberfläche fokussiert wird. Der fokussierte Laserstrahl 35 wird an einer im Winkelstück 33 angeordneten Umlenkvorrichtung, wie einem Spiegel 36, um 90° umgelenkt und auf die Gewebeoberfläche fokussiert. Von der Gewebeoberfläche werden optische Signale wie eine Fluoreszenzstrahlung oder eine SHG-(Second Harmonic Generation) Strahlung zurückgeworfen, wobei der Strahlengang der optischen Signale zunächst im Wesentlichen dem Strahlengang des Laserstrahls 35 entspricht. Der Strahlengang der optischen Signale verläuft somit ebenso durch die Linse 31, wird jedoch anschließend mittels dreier verschiedener optischer Erfassungsvorrichtungen 34 erfasst und detektiert. Jede der drei dargestellten optischen Erfassungsvorrichtungen 34 weist jeweils eine Umlenkvorrichtung 34.1, eine Linse 34.2 und einen optischen Sensor 34.3 auf. Die drei Umlenkvorrichtungen 34.1 können jeweils als Spiegel oder Umlenkprismen ausgebildet sein, die jeweils nur bestimmte Wellenlängenbereiche der zurückgeworfenen Strahlung reflektieren bzw. transmittieren. So können beispielsweise auch simultan innerhalb einer Diagnoseprozedur sowohl die SHG-Strahlung als auch die Fluoreszenzstrahlung als auch die reflektierte Strahlung (etwa für eine OCT-(optische Kohärenztomographie) Messung) detektiert werden. Wahlweise können auch nur zwei der vorgenannten Strahlungen detektiert werden, während die verbliebene Erfassungsvorrichtung deaktiviert ist. Schließlich kann auch vorgesehen sein, dass innerhalb einer Diagnoseprozedur nur eine Strahlungsart detektiert wird, während die jeweiligen anderen optischen Erfassungsvorrichtungen 34 deaktiviert sind.

Die optischen Erfassungsvorrichtungen 34 können mit einer Auswerteeinrichtung (nicht dargestellt) verbunden sein, in der aufgrund der Intensität oder anderer Eigenschaften der zurückgeworfenen Strahlung auf bestimmte Eigenschaften des untersuchten Gewebes geschlossen wird, insbesondere im Falle eines Zahns, ob es sich um kariöses Zahnmaterial handelt. Falls die Auswertung auf der Basis von mehreren Diagnose- und Strahlungsarten durchgeführt wird, so kann beispielsweise vorgesehen sein, dass die Auswertung bezüglich aller Diagnose- und Strahlungsarten ergeben muss, dass Karies vorliegt, in welchem Fall ein Befehl zur Ablation der diagnostizierten Stelle durch die Auswerteeinrichtung abgegeben wird.

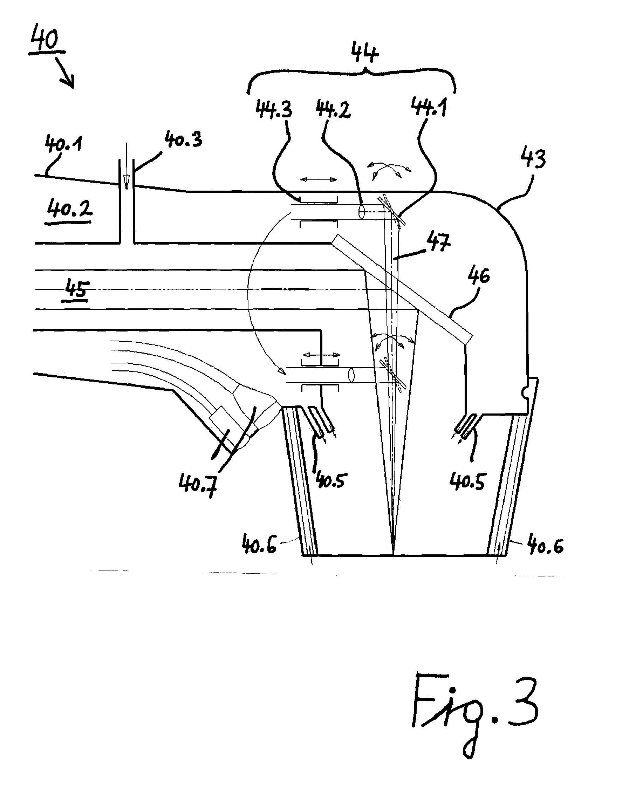

Die Fig. 3 zeigt eine schematische Querschnitts-Darstellung eines Endabschnitts eines Handstücks gemäß einer Ausführungsform. Das Handstück 40 weist eine Außenwand 40.1 auf, die einen Handstück-Innenraum 40.2 umschließt. An einen longitudinalen Abschnitt des Handstücks 40 schließt sich ein Winkelstück 43 an, in welchem eine Umlenkvorrichtung, wie ein Spiegel 46, einen in dem Handstück-Innenraum 40.2 geführten Laserstrahl 45 um einen Winkel von 90° in Richtung auf ein Handstückende umlenkt. Unterhalb des Handstückendes befindet sich eine zu untersuchende Gewebeoberfläche wie eine Zahnoberfläche. Der Laserstrahl 45 wird durch eine Linse (nicht dargestellt) fokussiert, sodass an einer Stirnseite des Handstückendes oder kurz darunter sich der Fokus des Laserstrahls 45 befindet. An der Stirnseite des Handstückendes kann wahlweise eine Abschlussscheibe angeordnet oder auch weggelassen sein. Durch eine Zuführung 40.3 kann zudem ein Medium in den Handstück-Innenraum 40.2 geleitet werden oder ein Vakuum in dem Handstück-Innenraum 40.2 erzeugt werden.

Bei einer Diagnose wird ein Diagnose-Laserstrahl 45 oder eine andere geeignete monochromatische oder auch breitbandige Strahlung auf die Gewebeoberfläche gerichtet. Von dieser wird ein Strahlungsbündel 47 zurückgeworfen oder reflektiert, welches zu erfassen und zu detektieren ist. Zu diesem Zweck ist innerhalb des Handstück-Innenraums 40.2 eine optische Erfassungsvorrichtung 44 angeordnet, die um die Längsachse des Handstücks 40 verschwenkt werden kann (langer gebogener Pfeil) und in der Darstellung der Fig. 3 an zwei verschiedenen Positionen gezeigt ist. Die optische Erfassungsvorrichtung 44 kann beispielsweise in einer revolverartigen Schwenkvorrichtung (nicht dargestellt) montiert werden und somit durch Drehen der revolverartigen Schwenkvorrichtung um die Längsachse des Handstücks von einer oberen Position in eine untere Position verschwenkt werden.

Die optische Erfassungsvorrichtung 44 enthält eine Umlenkvorrichtung wie einen Spiegel 44.1, eine Linse 44.2 und einen Detektor 44.3, wobei die Einheit bestehend aus Linse 44.2 und Detektor 44.3 linear entlang der Strahlrichtung der zu detektierenden Strahlung verschiebbar angeordnet ist (kurze Doppelpfeile), um solchermaßen eine Autofokusfunktion bereitzustellen. Der Spiegel 44.1 ist um eine durch seinen Schwerpunkt verlaufenden Achse senkrecht zur Papierebene und eine dazu senkrechte Achse verschwenkbar (kurze gebogene Doppelpfeile), sodass er die von verschiedenen Punkten der Gewebeoberfläche zurückgeworfene oder reflektierte Strahlung erfassen und in Richtung auf den Detektor 44.3 weiterleiten kann. Der Laserstrahl 45 kann durch eine Scan-Einheit (nicht dargestellt) räumlich über die Gewebeoberfläche gescannt werden, um so verschiedene Punkte der Gewebeoberfläche abzurastern. Im gleichen Maße kann der Spiegel 44.1 dem Laserstrahl 45 bei seiner abrasternden Bewegung folgen, indem er stets auf dem Punkt der Gewebeoberfläche ausgerichtet ist, den der Laserstrahl 45 soeben überstreift.

Die revolverartige Schwenkvorrichtung ist mechanisch so auszuführen, dass beim Verschwenken der Erfassungsvorrichtung 44 um die Längsachse des Handstücks 40 der Spiegel 44.1 stets nach unten zur Gewebeoberfläche zeigt. Dies kann beispielsweise dadurch ermöglicht werden, dass bei der Verschwenkung eine Gegenrotation des Spiegels 44.1 erfolgt, etwa durch einen Zapfen, der in eine Führungsnut eingreift wie in einer Schaltwalze eines Motorradgetriebes.

Für den Fall, dass sich die optische Erfassungsvorrichtung 44 in der oberen Position befindet, ist der Spiegel 46 für die von dem Detektor 44.3 zu erfassende Strahlung durchlässig. Falls sich die optische Erfassungsvorrichtung 44 jedoch in der unteren Position befindet, so kann vorgesehen sein, dass der Spiegel 44.1 für die Wellenlänge des Laserstrahls 45 durchlässig ist, um keine Absorption des Laserstrahls 45 an dem Spiegel 44.1 und damit unnötigen Lichtverlust zu erzeugen. Es ist ebenso möglich, an der unteren und an der oberen Position der revolverartigen Schwenkvorrichtung jeweils eine eigene optische Erfassungsvorrichtung 44 vorzusehen, welche unterschiedliche Wellenlängen oder Wellenlängenbereiche der zurückgeworfenen oder reflektierten Strahlung erfasst und detektiert. So kann beispielsweise eine an dem unteren Punkt der revolverartigen Schwenkvorrichtung montierte optische Erfassungsvorrichtung 44 die SHG-Strahlung erfassen und detektieren, während eine an der oberen Position der revolverartigen Schwenkvorrichtung montierte optische Erfassungsvorrichtung 44 eine Fluoreszenzstrahlung erfasst und detektiert. Im Falle einer derartigen Anordnung wäre dann der Spiegel 44.1 der unteren optischen Erfassungsvorrichtung 44 für die Wellenlänge der Fluoreszenzstrahlung als durchlässig vorzusehen.

Das Handstück 40 kann des Weiteren noch düsenartige Vorrichtungen 40.5 aufweisen, mit welchen geeignete Medien in Richtung auf die Gewebeoberfläche transportiert werden können. Des Weiteren kann das Handstück 40 am Handstückende eine Doppelwand 40.6 aufweisen, die dazu verwendet werden kann, als Absaugkanal zu dienen, um somit beispielsweise ablatiertes Zahnmaterial oder dergleichen effizient abzusaugen. Das Handstück 40 kann ferner Lichtwellenleiter 40.7 aufweisen, die entweder beispielsweise zur Beleuchtung der Gewebeoberfläche verwendet werden können oder mittels derer andere optische Signale zu- oder abgeführt werden können.

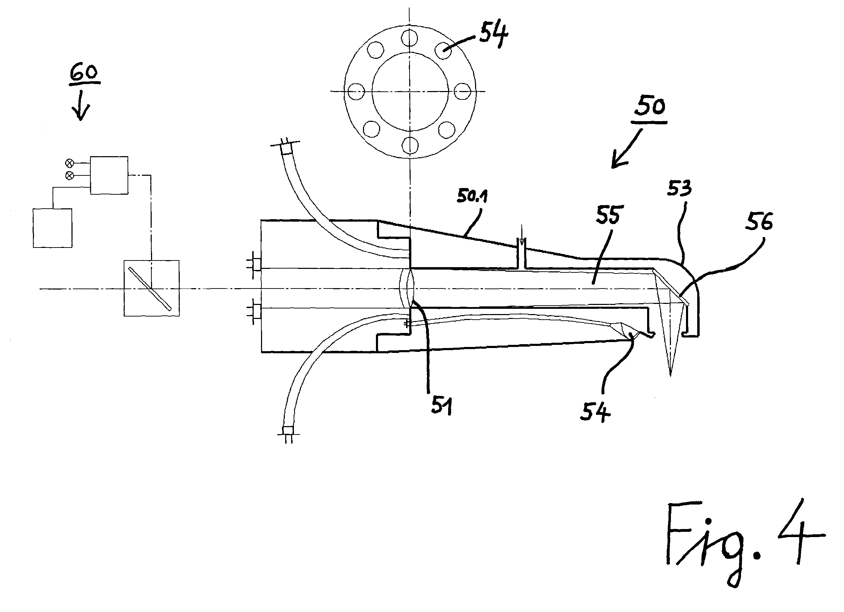

Die Fig. 4 zeigt eine schematische Querschnitts-Darstellung eines Handstücks gemäß einer Ausführungsform und eine Blockdarstellung einer daran angeschlossenen Auswerteeinrichtung. Das Handstück 50 weist einen longitudinalen Abschnitt mit einer Gehäusewandung 50.1 auf. Im Endbereich des longitudinalen Abschnitts ist ein Winkelstück 53 vorgesehen, innerhalb dessen ein Spiegel 56 angeordnet ist, mit dem ein Laserstrahl 55 aus einer Längsachse um 90° umgelenkt werden kann. Das Handstück 50 weist ferner eine optische Erfassungsvorrichtung in Form eines Abschnitts eines Lichtwellenleiters 54 auf, welcher auf eine Gewebeoberfläche ausgerichtet ist, auf welche der Laserstrahl 55 fokussiert wird. Mittels des Lichtwellenleiters 54 werden optische Signale von der Gewebeoberfläche erfasst und weitergeleitet.

Mittels einer Linse 51 wird der Laserstrahl 55 auf die Gewebeoberfläche fokussiert, wobei die Linse 51 linear in Strahlrichtung verschiebbar ist und von einer Autofokus-Steuereinrichtung angesteuert wird. Auf der Höhe der Linse 51 ist ein Querschnitt senkrecht zur Papierebene durch das Handstück 50 dargestellt. In diesem Querschnitt ist gezeigt, dass um den Umfang des kreisförmigen Querschnitts verteilt eine Mehrzahl von optischen Lichtwellenleitern 54 angeordnet ist. Einer dieser Lichtwellenleitern 54 ist Teil einer Kohärenztomographie-Einrichtung, deren Auswerteeinrichtung 60 in Blockdarstellung gezeigt ist. Der mit der OCT-Einrichtung verbundene Lichtwellenleiter 54 nimmt das reflektierte oder zurückgeworfene Signal von der Gewebeoberfläche auf und führt es der OCT-Einrichtung zu. Die übrigen Lichtwellenleiter 54 erfassen ebenfalls zurückgestreute oder reflektierte Strahlung von der Gewebeoberfläche und führen diese zu Fotodioden, mit welchen bestimmte Wellenlängenbereiche dieser Strahlung detektiert werden sollen. Auf diese Weise können bestimmte Wellenlängenbereiche im Spektrum der zurückgeworfenen Strahlung detektiert werden.

## Patentansprüche

1. Handstück (40) für ein medizinisches Diagnosegerät, mit einem Handstück-Innenraum (40.2), in welchem eine Strahlung in Richtung auf ein Handstückende führbar ist,
**gekennzeichnet durch**
eine oder mehrere optische Erfassungsvorrichtungen (44; 54), welche jeweils mindestens teilweise im Handstück-Innenraum (40.2) angeordnet sind.

2. Handstück (40) nach Anspruch 1,
**gekennzeichnet durch**
eine im Handstück-Innenraum (40.2) angeordnete erste Umlenkvorrichtung (46) zur Umlenkung eines Laserstrahls (45) in Richtung auf das Handstückende.

3. Handstück (40) nach Anspruch 1 oder 2,
**dadurch gekennzeichnet, dass**
die optische Erfassungsvorrichtung (44; 54) für die Erfassung von am Handstückende in den Handstück-Innenraum (40.2) eintretenden optischen Signalen ausgelegt ist.

4. Handstück (40) nach Anspruch 3,
**dadurch gekennzeichnet, dass**
die optische Erfassungsvorrichtung (44; 54) für die Erfassung und Detektion von optischen Signalen eines ersten vorgegebenen Wellenlängenbereichs ausgelegt ist.

5. Handstück (40) nach Anspruch 3 oder 4,
**dadurch gekennzeichnet, dass**
die optische Erfassungsvorrichtung (44) in einem Strahlengang der optischen Signale eine zweite Umlenkvorrichtung (44.1), eine Linse (44.2) und einen optischen Sensor (44.3) aufweist.

6. Handstück (40) nach Anspruch 5,
**dadurch gekennzeichnet, dass**
eine die Linse (44.2) und den optischen Sensor (44.3) aufweisende Einheit in einer Strahlrichtung der optischen Signale linear verschiebbar ausgelegt ist.

7. Handstück (40) nach Anspruch 5 oder 6,
**dadurch gekennzeichnet, dass**
die zweite Umlenkvorrichtung (44.1) um eine durch die zweite Umlenkvorrichtung (44.1) hindurchtretende Achse schwenkbar gehaltert ist, insbesondere um zwei aufeinander senkrechte, durch die zweite Umlenkvorrichtung (44.1) hindurchtretende Achsen schwenkbar gehaltert ist.

8. Handstück (40) nach einem der Ansprüche 3 bis 7,
**dadurch gekennzeichnet, dass**
die optische Erfassungsvorrichtung (44) im Strahlengang der optischen Signale nach der ersten Umlenkvorrichtung (46) angeordnet oder anordenbar ist und die erste Umlenkvorrichtung (46) für die Strahlung der optischen Signale durchlässig ist.

9. Handstück (40) nach einem der vorhergehenden Ansprüche,
**dadurch gekennzeichnet, dass**
die erste Umlenkvorrichtung (46) zur Umlenkung des Laserstrahls aus einer Längsachse des Handstücks (40) in einer Querachse ausgelegt ist, und
die optische Erfassungsvorrichtung (44) um die Längsachse schwenkbar ist.

10. Handstück (40) nach Anspruch 9,
**dadurch gekennzeichnet, dass**
die optische Erfassungsvorrichtung (44) an einer revolverartigen Schwenkvorrichtung befestigbar oder befestigt ist.

11. Handstück (40) nach einem der vorhergehenden,
**gekennzeichnet durch**
eine weitere optische Erfassungsvorrichtung, welche im Handstück-Innenraum angeordnet ist.

12. Handstück (40) nach den Ansprüchen 10 und 11,
**dadurch gekennzeichnet, dass**
die weitere optische Erfassungsvorrichtung an der revolverartigen Schwenkvorrichtung befestigbar oder befestig ist.

13. Handstück (40) nach den Ansprüchen 4 und 11,
**dadurch gekennzeichnet, dass**
die weitere optische Erfassungsvorrichtung für die Erfassung und Detektion von optischen Signalen eines von dem ersten vorgegebenen Wellenlängenbereich verschiedenen zweiten vorgegebenen Wellenlängenbereichs ausgelegt ist.

14. Handstück (40) nach einem der Ansprüche 1 bis 4,
**dadurch gekennzeichnet, dass**
die optische Erfassungsvorrichtung einen optischen Lichtwellenleiter (54) aufweist, welcher mit seinem lichteintrittsseitigen Ende auf das Handstückende ausgerichtet ist.

15. Medizinisches Diagnosegerät zur Erkennung von Karies an Zähnen, mit
einer oder mehreren Strahlungsquellen (11) zur Aussendung von Strahlung von jeweils einer vorgegebenen Wellenlänge auf einen zu diagnostizierenden Bereich (25), **gekennzeichnet durch**
ein Handstück (20) nach einem der vorhergehenden Ansprüche, und
eine Auswerteeinrichtung (12), welche mit der einen oder den mehreren optischen Erfassungsvorrichtungen verbunden ist.

16. Medizinisches Diagnosegerät nach Anspruch 15,
**dadurch gekennzeichnet, dass**
die Strahlungsquelle oder eine der Strahlungsquellen (11) eine Laserstrahlquelle aufweist und die optische Erfassungsvorrichtung einen optischen Sensor aufweist, welcher für die Detektion von von dem Gewebe (25) zurückgeworfener Strahlung der zweiten Harmonischen der Laserstrahlung ausgelegt ist.

17. Medizinisches Diagnosegerät nach Anspruch 15 oder 16,
**dadurch gekennzeichnet, dass**
die Strahlungsquelle (11) oder eine der Strahlungsquellen monochromatische Strahlung emittiert und die optische Erfassungsvorrichtung einen optischen Sensor aufweist, welcher für die Detektion von von dem Gewebe (25) zurückgeworfener Fluoreszenzstrahlung ausgelegt ist.

18. Medizinisches Diagnosegerät nach einem der Ansprüche 15 bis 17,
**dadurch gekennzeichnet, dass**
die optische Erfassungsvorrichtung einen optischen Sensor aufweist, welcher für die Detektion von UV-Strahlung ausgelegt ist.

19. Medizinisches Diagnosegerät nach einem der Ansprüche 15 bis 18,
**gekennzeichnet durch**
eine Kohärenztomographie-Einrichtung.

20. Medizinisches Diagnosegerät nach Anspruch 19,
**dadurch gekennzeichnet, dass**
die optische Erfassungsvorrichtung einen optischen Lichtwellenleiter aufweist, welcher ein Teil der Kohärenztomographie-Einrichtung ist und mit seinem lichteintrittsseitigen Ende auf das Handstückende ausgerichtet ist.
